(19) Europäisches Patentamt
European Patent Office
Office européen des brevets

(11) EP 1 275 722 A1

(12) # EUROPEAN PATENT APPLICATION

(43) Date of publication:
**15.01.2003 Bulletin 2003/03**

(21) Application number: **01610075.2**

(22) Date of filing: **11.07.2001**

(51) Int Cl.7: **C12N 15/55**, C12N 9/16, C12Q 1/68, C12Q 1/48, A01K 67/027

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **NOVO NORDISK A/S**
**2880 Bagsvaerd (DK)**

(72) Inventors:
- **Echwald, Soren Morgenthaler**
  **3050 Humlebaek (DK)**
- **Sondergaard, Helle Bach**
  **2100 Kobenhavn (DK)**
- **Pedersen, Oluf Borbye**
  **2800 Lyngby (DK)**

(54) **A P387L variant in protein tyrosine phosphatase-1B is associated with type 2 diabetes and impaired serine phosphorylation of PTP-1B in vitro**

(57) The present invention provides an isolated polynucleotide molecule comprising a nucleotide sequence encoding PTP-1B, said nucleotide sequence containing a mutation associated with type 2 diabetes of at least one nucleotide, or comprising a fragment of the nucleotide sequence including said mutation.

EP 1 275 722 A1

## Description

### FIELD OF THE INVENTION

The present invention relates to a mutant DNA sequence encoding protein tyrosine phosphatase-1B (PTP-1B), a method of detecting a mutation in the gene encoding protein tyrosine phosphatase-1B, as well as a diagnostic composition and a test kit for use in the method.

### BACKGROUND OF THE INVENTION

Type 2 diabetes, also known as non-insulin dependent diabetes mellitus (NIDDM), is one of the most common of all metabolic disorders and poses a major health problem worldwide. Type 2 diabetes results from defects in both insulin secretion and insulin action, but the exact underlying mechanism(s) causing the disease are not known. An elevation of hepatic glucose production contributes significantly to causing fasting hyperglycemia, whereas decreased insulin-mediated glucose uptake by muscle and fat is a major contributor to postprandial hyperglycemia. Moreover, the metabolic fate of glucose taken up by muscle is not normal in people with type 2 diabetes. For example muscle glycogen synthase activity and glycogen synthesis have been shown to be impaired in type 2 diabetes. The available treatments do not allow for a complete normalisation of the metabolic state and some of them are associated with side effects. The metabolic derangements created by hyperglycemia, together with the strong association between type 2 diabetes, obesity, hypertension, and hyperlipidemia, lead to an extensive list of long-term complications, including a high rate of cardiovascular death due to accelerated atherosclerosis, as well as typical complications of diabetes such as retinopathy, nephropathy, and neuropathy.

There is extensive circumstantial evidence from family investigations including studies in twins and from studies of hybrid populations descended from high- and low-risk ancestral populations in favour of genetic determinants for the common late onset form of type 2 diabetes. It is also likely that type 2 diabetes in many cases is polygenic and it is suggested that subsets of patients display changes in various diabetes susceptibility genes thereby adding to the heterogeneity of type 2 diabetes.

As the symptoms of type 2 diabetes usually occur up to years after the onset of the disease and as type 2 diabetes is often first diagnosed when the long-term complications appear, there is a strong need for methods which enable an earlier diagnosis of type 2 diabetes. One such method could involve the detection of such genetic determinants associated with susceptibility for developing type 2 diabetes.

### SUMMARY OF THE INVENTION

According to the present invention, it has now been found that variability in the *PTP-1b* (GenBank Accession Number M31724) gene (SEQ ID NO: 1, the polypeptide encoding part of SEQ ID NO: 1 is situated from nucleotide 91 to nucleotide 1398, including start and stop codons), also known as PTPN1, confers susceptibility to type 2 diabetes and that a widespread missense polymorphism of this gene is reproducibly associated with type 2 diabetes. One or more of such mutations may be involved in or associated with the etiology of type 2 diabetes, and their presence may therefore be diagnostic for type 2 diabetes and possibly also other disorders associated with type 2 diabetes, like obesity, hyperlipidemia and hypertension. Without wishing to be bound by any theory, the mutation in PTP-1B associated with type 2 diabetes may be indicative of abnormalities significant for the development of type 2 diabetes or other disorders associated with type 2 diabetes. The mutation may for instance give rise to the substitution of an amino acid in PTP-1B that may cause changes in the tertiary structure of PTP-1B. Such changes may interfere with the normal interaction between PTP-1 B and the molecules with which it interacts. Mutations may also interfere with the post-translational processing of PTP-1 B often resulting in a PTP-1 B with an aberrant function. Mutations may also interfere with the transcription or translation of the gene, or with the stability of the PTP-1B transcript. Mutations may also cause defects in splicing of the gene. Alternatively, the mutation may be associated with (i.e. genetically linked with) the mutation or mutations, which causes the disease.

The variability of the gene may be used as a diagnostic tool to identify subjects who are at an increased risk of developing type 2 diabetes. The variant may also identify subjects with variable response to drugs which act via the peroxisome proliferator-activated receptor-γ or which act via PTP-1 b, such as PTPase inhibitors, in other words the variant might be useful for tailoring of antidiabetic medication. The variant may also point to a new gene, which could be of importance for development of new drugs.

Accordingly, the present invention encompasses an isolated polynucleotide molecule comprising a nucleotide sequence encoding PTP-1 B, said nucleotide sequence containing a mutation associated with type 2 diabetes of a nucleotide, or comprising a fragment of the nucleotide sequence including said mutation.

The present invention also encompasses a recombinant vector, especially an expression vector, comprising a polynucleotide according to the present invention.

The present invention also encompasses a cell line or a transgenic non-human mammal containing a polynucleotide according to the present invention or a recombinant vector according to the present invention.

The present invention also encompasses a method of detecting the presence of a mutation in the gene encoding PTP-1 B, the method comprising obtaining a biological sample from a subject and analysing the sample for a mutation associated with type 2 diabetes of at least one nucleotide in the *PTP-1B* sequence.

The present invention also encompasses a diagnostic composition for determining predisposition to type 2 diabetes in a subject, the composition comprising a polynucleotide according to the present invention.

The present invention also encompasses a test kit for detecting the presence of a mutation associated with type 2 diabetes in the gene encoding PTP-1 B, the kit comprising a first polynucleotide comprising a nucleotide sequence corresponding to at least part of the gene encoding PTP-1 B and containing a mutation of at least one nucleotide, which mutation corresponds to the mutation the presence of which in the gene encoding PTP-1 B is to be detected and optionally a second polynucleotide comprising a nucleotide sequence corresponding to at least part of the wild-type gene encoding PTP-1 B and/or optionally a restriction endonuclease, which cleaves DNA at the site of the mutation.

The present invention also encompasses a test kit for detecting the presence of a mutation associated with type 2 diabetes in the gene encoding PTP-1 B, the kit comprising means for amplifying DNA, and a labelled polynucleotide comprising a nucleotide sequence corresponding to at least part of the gene encoding PTP-1 B and containing a mutation of at least one nucleotide, which mutation corresponds to the mutation the presence of which in the gene encoding PTP-1 B is to be detected.

The present invention also encompasses an isolated polypeptide obtainable by expression of a DNA construct comprising a polynucleotide according to the present invention, where said mutation gives rise to an amino acid substitution in PTP-1 B.

The present invention also encompasses an isolated polypeptide, which is a variant of PTP-1 B carrying an amino acid substitution associated with type 2 diabetes and which variant is selected from the group consisting of (a) a polypeptide having an amino acid sequence which substantially homologous to residues 1 to 435 of SEQ ID NO: 2; (b) a polypeptide which is encoded by a polynucleotide comprising a nucleic acid sequence which hybridizes under low stringency conditions with (i) nucleotides 91 to 1395 of SEQ ID NO: 1 or (ii) a subsequence of (i) of at least 100 nucleotides, (c) a variant of a polypeptide comprising an amino acid sequence of SEQ ID NO: 2 comprising a substitution, deletion, and/or insertion of one or more amino acids; (d) an allelic variant of (a) or (b); and (e) a fragment of (a), (b), (c) or (d).

Further embodiments will become apparent from the following detailed description.

## FIGURES AND TABLES

**Figure 1:** *in vitro* peptide serine phosphorylation by $p34^{cdc2}$ protein kinase. Equal amounts of wild type and mutant peptide were loaded. Visualized radioactive incorporation into wild type (387P) and mutant (387L) peptide after gel exposure to a phosphoimager screen. Lane 1-3 contains 25 µl 387P reaction, lane 4 15 µl BenchMark™ protein marker, lane 5-7 25 µl 387L reaction, lane 8 25 µl myelin basic protein (MBP)(positive control), lane 9 25 µl reaction mixture without $p34^{cdc2}$ kinase added and with MBP as substrate (negative control). The gel represents one independent assay with three replicates.

**Figure** 2: Percentage incorporation of [$Y$-$^{32}$p] ATP by the $p32^{cdc2}$ kinase into wild type peptides (RRRGAQAASPAKGE: 387P) and mutant peptides (RRRGAQAASLAKGE: 387L). The figure represents a mean of four independent *in vitro* peptide phosphorylation assays corrected for background. The wild type from each assay is 100% and the mutant is counted as a percentage of the wild type, 387P = 100% and 387L = 28.4% ± 5.8. (SD) Standard deviation is calculated from the log10-transformed data set.

Table 1: "Blosum 62" scoring matrix using a gap opening penalty of 10, a gap extension penalty of 1 for alignment of amino acid sequences (amino acids are indicated by the standard one-letter codes).

**Table 2:** Conservative amino acid substitutions.

**Table 3:** Nucleotide sequences of cDNA primers used for PCR amplification of the *PTP-1B* gene segments for SSCP-heteroduplex gel analyses and sequencing of variants: Nucleotide numbers (according to SEQ ID NO: 1) of the first nucleotide (5') in each primer are given in parentheses.

**Table 4:** Genotype association studies of the G381S, P387L, and 3'UTR+104insG variants in PTP-1B and allelic frequencies in type 2 diabetic patients and control subjects.

**Table 5:** Clinical and biochemical characteristics of type 2 diabetic patients classified according to genotype of the P387L variant of the *PTP-1B* gene.

## DEFINITIONS AND ABBREVIATIONS

"Corresponding to", when used in reference to a nucleotide or amino acid sequence, indicate the position in the second sequence that aligns with the reference position when two sequences are optimally aligned.

The term "isolated", when applied to a polynucleotide, denotes that the polynucleotide is removed from its natural genetic milieu. Such isolated molecules are those that are separated from the natural environment and include cDNA and genomic clones. When applied to a protein, the term "isolated" indicates that the polypeptide is found in a condition other than its native environment, such as apart from blood and animal tissue. It may also indicate that the polypeptide is chemically synthesized. The isolated polypeptide may be substantially free of other polypeptides, particularly other proteins of animal or plant origin. The polypeptide may be for instance at least about 20% pure, or at least about 40% pure, or at least about 60% pure, or at least about 80% pure, or at least about 90% pure, or at least about 95% pure or at least about 99% pure, as determined for instance by SDS-PAGE.

A "polynucleotide" is a single- or doublestranded polymer of nucleotides such as deoxyribonucleotide or ribonucleotide bases linked together by phosphodiester (5'-3') bonds and read from the 5' to the 3' end. Polynucleotides include RNA and DNA, and may be isolated from natural sources (including genetically engineered organisms), synthesized *in vitro,* or prepared form a combination of natural and synthetic molecules. It will be recognized by those skilled in the art that the two strands of a double-stranded polynucleotide may differ slightly in length and that the ends thereof may be staggered as a result of enzymatic cleavage; thus all nucleotides within a double-stranded molecule may not be paired. As the skilled person will recognize, this definition of a polynucleotide also comprises what is known as an oligonucleotide that is a polynucleotide containing a small number of nucleotides, such as for instance 9, 12, 15, 18, 21, 24, 27, 30 or 33 nucleotides. The polynucleotides of present invention also encompass DNA analogues, such as PNA and LNA.

A "DNA construct" is a polynucleotide as defined above as a single- or doublestranded polymer of deoxyribonucleotide bases.

A "polypeptide" is a linear polymer of amino acids held together by peptide linkages. The polypeptide has a directional sense with an amino and a carboxy terminal end. A polypeptide may be isolated from natural sources (including genetically engineered organisms), synthesized *in vitro,* or prepared form a combination of natural and synthetic molecules. As the skilled person will recognize, this definition of a polypeptide also comprises what is known as a peptide.

"Significant" is used in the context of the present invention to describe the result of a statistical analysis, where the statistical value p is lower than 0.05.

"Operably connected", when referring to DNA segments, indicates that the segments are arranged so that they function in concert for their intended purposes, e.g. transcription initiates in the promoter and proceeds through the coding segment to the terminator.

The abbreviations used are: PTP-1B, protein tyrosine phosphatase 1b; SSCP, single-strand conformation polymorphism; PCR, polymerase chain reaction; $p34^{cdc2}$ kinase, p34 cell-division-cycle kinase; MBP, myelin basic protein; OHA, oral hyperglycaemia agents, bp, base pair.

## DETAILED DESCRIPTION OF THE INVENTION

The present invention encompasses an isolated polynucleotide molecule comprising a nucleotide sequence encoding PTP-1B, said nucleotide sequence containing a mutation associated with type 2 diabetes of a nucleotide, or comprising a fragment of the nucleotide sequence including said mutation.

A polynucleotide molecule comprising a nucleotide sequence encoding PTP-1B also encompasses a polynucleotide which comprises a nucleotide sequence which is substantially homologous to the nucleotide sequence covering nucleotides 91 to 1395 of SEQ ID NO: 1 or a fragment thereof. The term "substantially homologous" is used herein to denote polynucleotides having a sequence identity to the sequence covering nucleotides 91 to 1395 shown in SEQ ID NO: 1 of at least about 65%, or at least about 70%, or at least about 80%, or at least about 90%, or at least about 95%, or at least about 97% while still encoding a polypeptide having an amino acid sequence substantially homologous to residues 1 to 435 of SEQ ID NO: 2. How to determine sequence identity of polynucleotide molecules is described further below.

SEQ ID NO: 1 is the wild-type DNA sequence coding for PTP-1 B (GenBank Accession Number M31724) and SEQ ID NO: 2 is the wild-type amino acid sequence of PTP-1B. Those skilled in the art will recognize that the DNA sequence in SEQ ID NO: 1 also provides the RNA sequence encoding SEQ ID NO: 2 by substituting U for T. Those skilled in the art will also readily recognise that, in view of the degeneracy of the genetic code, considerable sequence variation is possible among the polynucleotide molecules according to the present invention. Furthermore, the polynucleotide molecules according to the present invention may contain other sequence variations corresponding to amino acid substitutions in SEQ ID NO: 2 as long as this does not interfere with the utility of the polynucleotides according to the purpose of the present invention. Such sequence variations could for instance correspond to a genetic variation, such as in the form of an allelic variant, within a specific population, a member of which is being diagnosed for susceptibility for developing type 2 diabetes or other disorders associated with type 2 diabetes, like obesity, hyperlipidemia and hypertension, but could also be related to other amino acid substitutions of interest.

Those skilled in the art will also recognize that a polynucleotide according to the present invention or a fragment of a polynucleotide according to the present invention may also contain more than one mutation associated with type 2 diabetes or other disorders associated with type 2 diabetes or indeed other mutations of interest.

The length of the polynucleotides according to the present invention may vary widely depending on the intended use. For use as a polynucleotide probe for hybridisation purposes, the polynucleotide may be as short as for instance 17 nucleotides or even shorter. For expression in a cell line or a transgenic non-human mammal as defined above, the polynucleotide according to the present invention will typically comprise the full-length DNA sequence encoding PTP-1B. For instance for use in PCR reactions a polynucleotide according to the present invention may comprise additional nucleotides in the N-terminal such as nucleotides forming a restriction endonuclease site for subsequent digestion and cleaving.

The polynucleotide of the present invention comprising the mutation in the nucleotide sequence encoding PTP-1B may suitably be of genomic DNA or cDNA origin, for instance obtained by preparing a genomic or cDNA library and screening for DNA sequences coding for all or part of the PTP-1 B by hybridisation using synthetic oligonucleotide probes in accordance with standard techniques (cf. Sambrook et al., Molecular Cloning: A Laboratory Manual, 2nd Ed., Cold Spring Harbor, Cold Spring Harbor Laboratory Press, 1989). The probes used should be specific for the mutation. Alternatively, the DNA molecule encoding wild-type PTP-1B may be modified by site-directed mutagenesis using synthetic oligonucleotides containing the mutation for homologous recombination in accordance with well-known procedures. The polynucleotides, especially the DNA constructs, according to the present invention, may also be prepared by polymerase chain reaction using specific primers, for instance as described in US 4,683,202, or Saiki et al., Science 239, 487-491 (1988).

The polynucleotides, especially the DNA constructs, of the present invention may also be prepared synthetically by established standard methods, e.g. the phosphoamidite method described by Beaucage and Caruthers, Tetrahedron Letters 22, 1859-1869 (1981), or the method described by Matthes et al., EMBO Journal 3, 801-805 (1984). According to the phosphoamidite method, oligonucleotides are synthesized, e.g. in an automatic DNA synthesizer, purified, annealed and ligated. This procedure may preferably be used to prepare fragments of the PTP-1B encoding DNA sequence.

In one embodiment, the present invention encompasses a polynucleotide according to the present invention comprising a nucleotide sequence as shown in the Sequence Listing as SEQ ID NO: 1 containing a mutation associated with type 2 diabetes of at least one nucleotide or comprising a fragment of the nucleotide sequence shown in the Sequence Listing as SEQ ID NO: 1 including said mutation.

In another embodiment, the present invention encompasses a polynucleotide according to the present invention, where said mutation gives rise to an amino acid substitution in PTP-1 B.

In another embodiment, the present invention encompasses a polynucleotide according to the present invention, where a polypeptide encoded by said first polynucleotide in a $p34^{cdc2}$ kinase phosphorylation assay has a degree of phosphorylation in a $p34^{cdc2}$ kinase phosphorylation assay which is significantly lower than the degree of phosphorylation of a second polypeptide encoded by a polynucleotide which differs from the first polynucleotide only by not containing said mutation associated with type 2 diabetes.

In a further embodiment, the present invention encompasses a polynucleotide according to the present invention, where said phosphorylation takes place at the serine residue in the position corresponding to corresponding to position 386 of the amino acid sequence shown in the Sequence Listing as SEQ ID NO: 2.

In another embodiment, the present invention encompasses a polynucleotide according to the present invention, where said mutation gives rise to a substitution of Pro to an amino acid different from Pro in the position corresponding to position 387 of the amino acid sequence shown in the Sequence Listing as SEQ ID NO: 2.

In another embodiment, the present invention encompasses a polynucleotide according to the present invention, where said mutation corresponds to a mutation of C in position 1250 in SEQ ID NO: 1 to T.

In another embodiment, the present invention encompasses a polynucleotide according to the present invention wherein said polynucleotide is a DNA construct.

The present invention also encompasses a recombinant vector, especially an expression vector, comprising a polynucleotide according to the present invention.

The recombinant vector into which a polynucleotide according to the present invention is inserted may be any vector that conveniently may be subjected to recombinant DNA procedures. The choice of vector will often depend on the host cell into which it is to be introduced. Thus, the vector may be an autonomously replicating vector, i.e. a vector that exists as an extrachromosomal entity, the replication of which is independent of chromosomal replication, e.g. a plasmide. Alternatively, the vector may be one which, when introduced into a host cell, is integrated into the host cell genome and replicated together with the chromosome(s) into which it has been integrated (e.g. a viral vector).

In the vector, the mutant DNA sequence encoding PTP-1B may be operably connected to a suitable promoter sequence. The promoter may be any DNA sequence, which shows transcriptional activity in the host cell of choice and may be derived from genes encoding proteins either homologous or heterologous to the host cell. Examples of suitable promoters for directing the transcription of the mutant DNA encoding PTP-1 B in mammalian cells are the SV40 promoter (Subramani et al., Mol. Cell Biol. 1, 854 -864 (1981)), the MT-1 (metallothionein gene) promoter (Palmiter et al., Science 222, 809-814 (1983)) or the adenovirus 2 major late promoter.

The mutant DNA sequence encoding PTP-1B may also be operably connected to a suitable terminator, such as the human growth hormone terminator (Palmiter et al., ibid.). The vector may further comprise elements such as polyadenylation signals (e.g. from SV40 or the adenovirus 5 Elb region), transcriptional enhancer sequences (e.g. the SV40 enhancer) and translational enhancer sequences (e.g. the ones encoding adenovirus VA RNAs).

The recombinant expression vector may further comprise a DNA sequence enabling the vector to replicate in the host cell in question. An example of such a sequence is the SV40 origin of replication. The vector may also comprise a selectable marker, e.g. a gene the product of which complements a defect in the host cell, such as the gene coding for dihydrofolate reductase (DHFR) or one which confers resistance to a drug, e.g. neomycin, hygromycin or methotrexate.

The procedures used to ligate the DNA sequences coding for PTP-1 B, the promoter and the terminator, respectively, and to insert them into suitable vectors containing the information necessary for replication, are well known to persons skilled in the art (cf., for instance, Sambrook et al. ibid.).

The present invention also encompasses a cell line or a transgenic non-human mammal containing a polynucleotide according to the present invention or a recombinant vector according to the present invention.

A cell line into which a polynucleotide or a recombinant vector according to the present invention may be introduced may be any cell in which the polynucleotide can be replicated, such as a prokaryotic cell, for example *Eschericia coli* or a eukaryotic cell, such as a vertebrate cell, e.g. a *Xenopus laevis* oocyte or a mammalian cell. The cell line into which a polynucleotide according to the present invention is introduced may also be a cell which is capable of producing PTP-1B and which has the appropriate signal transduction pathways. Such a cell is preferably a eukaryotic cell, such as a vertebrate cell, e.g. a *Xenopus laevis* oocyte or mammalian cell, in particular a mammalian cell. Examples of suitable mammalian cell lines are the COS (ATCC CRL 1650), BHK (ATCC CRL 1632, ATCC CCL 10), CHL (ATCC CCL39) or CHO (ATCC CCL 61) cell lines.

Methods of transfecting cells, such as prokaryotic cells and eukaryotic cells, such as mammalian cells, are described in e.g. Old RW, Primrose SB: Principles of gene manipulation - an introduction to genetic engineering. Fifth edition. Blackwell Science Ltd. Oxford, 1994.

Expressing DNA sequences introduced in such cells especially eukaryotic cells, and more especially mammalian cells, are described in e.g. Kaufman and Sharp, J. Mol. Biol. 159, 601-621 (1982); Southern and Berg, J. Mol. Appl. Genet. 1, 327-341 (1982); Loyter et al., Proc. Natl. Acad. Sci. USA 79, 422-426 (1982); Wigler et al., Cell 14, 725 (1978); Corsaro and Pearson, Somatic Cell Genetics 7, 603 (1981); Graham and van der Eb, Virology 52, 456 (1973); and Neumann et al., EMBO J. 1, 841-845 (1982).

A mutant DNA sequence encoding PTP-1B may then be expressed by culturing cells as described above in a suitable nutrient medium under conditions, which are conducive to the expression of the PTP-1B-encoding DNA sequence. The medium used to culture the cells may be any conventional medium suitable for growing such a cell, such as medium suitable for growing mammalian cells, such as a serum-containing or serum-free medium containing appropriate supplements. Suitable media are available from commercial suppliers or may be prepared according to published recipes (e.g. in catalogues of the American Type Culture Collection).

A polynucleotide according to the present invention may also be introduced into a transgenic animal. A transgenic animal is one in whose genome a heterologous DNA sequence has been introduced. In particular, the transgenic animal is a transgenic non-human mammal, mammals being generally provided with appropriate signal transduction pathways. The mammal may conveniently be a rodent such as a rat or mouse. The mutant DNA sequence encoding PTP-1B may be introduced into the transgenic animal by any one of the methods previously described for this purpose. Briefly, the DNA sequence to be introduced may be injected into a fertilised ovum or cell of an embryo, which is subsequently implanted into a female mammal by standard methods, resulting in a transgenic mammal whose germ cells and/or somatic cells contain the mutant DNA sequence. For a more detailed description of a method of producing transgenic mammals, vide B. Hogan et al., Manipulating the Mouse Embryo, Cold Spring Harbor Laboratory, Cold Spring Harbor, New York. The mutant DNA sequence may also be introduced into the animal by transfection of fertilised ova with a retrovirus containing the DNA sequence, cf. R. Jaenisch (1976), Proc. Natl. Acad. Sci. USA 73, 1260-1264. A further method of preparing transgenic animals is described in Gordon and Ruddle, Methods Enzymol. 101, 411-432 (1983).

The present invention also encompasses a method of detecting the presence of a mutation in the gene encoding PTP-1B, the method comprising obtaining a biological sample from a subject and analysing the sample for a mutation associated with type 2 diabetes of a nucleotide in the *PTP-1B* sequence.

In one embodiment of the present invention a method according to the present invention comprises analysing said sample for a mutation associated with type 2 diabetes of a nucleotide in the *PTP-1B* sequence, which mutation gives rise to an amino acid substitution in PTP-1B.

In another embodiment of the present invention a method according to the present invention comprises analysing said sample for a mutation associated with type 2 diabetes of a nucleotide in the *PTP-1B* sequence, which mutation gives rise to a substitution of Pro to an amino acid different from Pro in the position corresponding to position 387 of the amino acid sequence shown in the Sequence Listing as SEQ ID NO: 2.

In another embodiment of the present invention a method according to the present invention comprises analysing said sample for a mutation associated with type 2 diabetes of a nucleotide in the *PTP-1B* sequence, which mutation corresponds to a mutation of C in position 1250 in SEQ ID NO: 1 to T.

Those skilled in the art will readily recognize that it is within the scope of the present invention to analyse said samples for more than one mutation in PTP-1 B associated with type 2 diabetes or other disorders associated with type 2 diabetes or additionally analyse said samples for other mutations in PTP-1B of interest, or indeed for mutations in other genes associated with diabetes or otherwise of interest.

In a further embodiment of a method according to the present invention, a biological sample is obtained from a subject, DNA (in particular genomic DNA) is isolated from the sample and digested with a restriction endonuclease which cleaves DNA at the site of the mutation, and cleavage of the DNA within the gene encoding PTP-1 B at this site is determined. After digestion, the resulting DNA fragments may be subjected to electrophoresis on an agarose gel. DNA from the gel may be visualised, for instance by staining with ethidium bromide. DNA from the gel may also be blotted onto a nitrocellulose filter and hybridised with a labelled probe, such as for instance a radiolabelled probe or a probe labelled as described further below. The probe may conveniently contain a DNA fragment of the PTP-1 B gene spanning the mutation (substantially according to the method of E.M. Southern, J. Mol. Biol. 98, 503 (1975), e.g. as described by B.J. Conner et al., Proc. Natl. Acad. Sci. USA 80, 278-282 (1983)).

Digestion of the DNA may preferably be performed as recommended by the supplier of the enzyme.

In a further embodiment of this method, the restriction pattern of the DNA after digestion with the restriction endonuclease, whether visualised by staining with ethidium bromide or by hybridising with a labelled probe or otherwise, is compared to the restriction pattern obtained with a negative control comprising at least a portion of wild-type DNA encoding PTP-1B (i.e. not containing the mutation) and/or to the restriction pattern obtained with a positive control comprising at least a portion of DNA encoding PTP-1 B and containing the mutation.

In a further embodiment of this method, the positive control comprises a polynucleotide according to the present invention.

In a further embodiment of a method according to the present invention the sample is analysed for a mutation associated with type 2 diabetes of a nucleotide in the *PTP-1B* sequence, which mutation corresponds to a mutation of C in position 1250 in SEQ ID NO: 1 to T, by isolating DNA from the sample and digesting it with a restriction endonuclease which cleaves DNA at the sequence

5' ...C C N N N N N/N N G G... 3'

3' ...G G N N/N N N N N C C... 5',

and determining cleavage of the DNA within the gene encoding PTP-1 B at this site as described above. / denotes the place of cleavage and N denotes any deoxyribonucleotide.

In a still further embodiment of this method, the restriction endonuclease is *Bs*/I.

In this case, the mutation removes a *Bs*/I restriction endonuclease site at position 101 in the segment. An additional control site is present at position 290 in the segment. Thus enzyme digestion of PCR segments from wildtype carriers will produce 3 fragments: 37bp, 101bp, and 189bp whereas homozygous mutant carriers will produce only the 37bp fragment and a 290bp fragment. Heterozygous carriers will contain all four fragments. Digestion may be performed as described under the heading "Genotyping".

It is readily recognized by those skilled in the art that other restriction endonucleases may be useful for analysing samples for other mutations not corresponding to a mutation of C in position 1250 in SEQ ID NO: 1 to T. It might even be conceived that a restriction endonuclease different from *Bs*/I also might be useful in this latter case. It is a question of routine work for a person skilled in the art to determine whether DNA spanning a given mutation associated with type 2 diabetes of a nucleotide in the *PTP-1B* sequence of interest may be cleaved by use of an restriction endonuclease and, in that case, which restriction endonuclease(s) will be suitable for the task and how to analyse the resulting restriction patterns.

In a variant of these embodiments, the DNA isolated from the sample may be amplified prior to digestion with the restriction endonuclease. Amplification may suitably be performed by polymerase chain reaction (PCR) using oligonucleotide primers based on the appropriate sequence of PTP-1 B spanning the site(s) of mutation, essentially as described by Saiki et al., Science 230, 1350-1354 (1985). After amplification, the amplified DNA may be digested with the appropriate restriction endonuclease and subjected to agarose gel electrophoresis. The restriction pattern obtained may be analysed, e.g. by staining with ethidium bromide and visualising bands in the gel by means of UV light. As a control, wild-type DNA encoding PTP-1B (i.e. not containing the mutation) may be subjected to the same procedure, and the restriction patterns may be compared.

In one embodiment of a method according to the present invention a biological sample is obtained from a subject, DNA is isolated from the sample, the DNA is amplified and hybridised to a labelled polynucleotide comprising a nucleotide sequence encoding PTP-1B, said nucleotide sequence containing a mutation associated with type 2 diabetes of at least one nucleotide, or comprising a fragment of the nucleotide sequence including said mutation, which mutation corresponds to the mutation the presence of which in the gene encoding PTP-1B is to be detected, and hybridisation of the labelled polynucleotide to the DNA is determined.

In a further embodiment of said method the labelled polynucleotide is a labelled polynucleotide according to the present invention.

In a further embodiment of said method, the amplified DNA is hybridised to a second labelled polynucleotide comprising a DNA sequence corresponding to at least part of the wild-type gene encoding PTP-1 B, and hybridisation of said second labelled polynucleotide to the amplified DNA is determined.

In a further embodiment of said method, the label substance with which the labelled polynucleotide carrying the mutation is labelled is different from the label substance with which the second labelled polynucleotide corresponding to at least part of the wild-type DNA is labelled.

The present invention also encompasses a method according to the present invention for determining predisposition to type 2 diabetes in a subject.

A further embodiment of a method according to the present invention is an adaptation of the method described by U. Landegren et al., Science 241, 1077-1080 (1988), which involves the ligation of adjacent oligonucleotides on a complementary target DNA molecule. Ligation will occur at the junction of the two oligonucleotides if the nucleotides are correctly base paired.

In a further embodiment of a method according to the present invention, the DNA isolated from the sample may be amplified using oligonucleotide primers corresponding to segments of the gene coding for PTP-1 B. The amplified DNA may then be analysed by hybridisation with a labelled polynucleotide comprising a DNA sequence corresponding to at least part of the gene encoding PTP-1 B and containing a mutation of at least one nucleotide, which mutation corresponds to the mutation the presence of which in the gene encoding PTP-1 B is to be detected. As a control, the amplified DNA may furthermore be hybridised with a further labelled polynucleotide comprising a DNA sequence corresponding to at least part of the wild-type gene encoding PTP-1 B. This procedure is, for instance, described by DiLella et al., Lancet 1, 497-499 (1988). Another PCR-based method which may be used in the present invention is the allele-specific PCR method described by R. Saiki et al., Nature 324, 163-166 (1986), or D.Y. Wu et al., Proc. Natl. Acad. Sci. USA 86, 2757-2760 (1989), which uses primers specific for the mutation in the *PTP-1B* gene.

Other methods of detecting mutations in DNA are reviewed in U. Landegren, GATA 9, 3-8 (1992). One of the currently preferred methods of detecting mutations is by single stranded conformation polymorphism (SSCP) analysis substantially as described by Orita et al., Proc. Natl. Acad. Sci. USA 86, 2766-2770 (1989), or Orita et al., Genomics 5, 874-879 (1989) and another is single base extension (also known as microsequencing) substantially as described by Syvänen, A.-C. et al., Genomics 12, 590-5 (1992).

The label substance with which a polynucleotide may be labelled may be selected from the group consisting of enzymes, coloured or fluorescent substances, or radioactive isotopes.

Examples of enzymes useful as label substances are peroxidases (such as horseradish peroxidase), phosphatases (such as acid or alkaline phosphatase), β-galactosidase, urease, glucose oxidase, carbonic anhydrase, acetylcholinesterase, glucoamylase, lysozyme, malate dehydrogenase, glucose-6-phosphate dehydrogenase, β-glucosidase, proteases, pyruvate decarboxylase, esterases, luciferase, etc.

Enzymes are not in themselves detectable but must be combined with a substrate to catalyse a reaction the end product of which is detectable. Examples of substrates, which may be employed in the method according to the invention, include hydrogen peroxide/tetramethylbenzidine or chloronaphthole or o-phenylenediamine or 3-(p-hydroxyphenyl) propionic acid or luminol, indoxyl phosphate, p-nitrophenylphosphate, nitrophenyl galactose, 4-methyl umbelliferyl-D-galactopyranoside, or luciferin.

Alternatively, the label substance may comprise coloured or fluorescent substances, including gold particles, coloured or fluorescent latex particles, dye particles, fluorescein, phycoerythrin or phycocyanin.

In one embodiment, the labelled polynucleotide is labelled with a radioactive isotope. Radioactive isotopes, which may be used for the present purpose, may be selected from I-125, I-131, In-111, H-3, P-32, C-14 or S-35. The radioactivity emitted by these isotopes may be measured in a beta- or gamma-counter or by a scintillation camera in a manner known per se.

The present invention also encompasses a diagnostic composition for determining predisposition to type 2 diabetes in a subject, the composition comprising a polynucleotide according to the present invention.

The present invention also encompasses a diagnostic composition for detecting the presence of a mutation in the gene encoding PTP-1B, the composition comprising a polynucleotide according to the present invention.

The present invention also encompasses a test kit for detecting the presence of a mutation associated with type 2 diabetes in the gene encoding PTP-1 B, the kit comprising a first polynucleotide comprising a nucleotide sequence corresponding to at least part of the gene encoding PTP-1B and containing a mutation of at least one nucleotide, which mutation corresponds to the mutation the presence of which in the gene encoding PTP-1B is to be detected and optionally a second polynucleotide comprising a nucleotide sequence corresponding to at least part of the wild-type gene encoding PTP-1 B and/or optionally a restriction endonuclease, which cleaves DNA at the site of the mutation.

In one embodiment of said test kit, the first polynucleotide is a polynucleotide according to the present invention.

In a further embodiment of said test kit, the first polynucleotide is a DNA construct, and said mutation corresponds to a mutation of C in position 1250 in SEQ ID NO: 1 to T and the test kit comprises a restriction endonuclease, which cleaves DNA at the site of the mutation.

In a still further embodiment of this test kit, the restriction endonuclease cleaves DNA at the sequence

5' ...C C N N N N N/N N G G... 3'

3' ...G G N N/N N N N N C C... 5'

/ denotes the place of cleavage and N denotes any deoxyribonucleotide.

In a still further embodiment of this test kit, the restriction endonuclease is *Bsl*I.

In another embodiment of the present invention, said test kit further comprises means for amplifying DNA.

The present invention also encompasses a test kit for detecting the presence of a mutation associated with type 2 diabetes in the gene encoding PTP-1 B, the kit comprising means for amplifying DNA, and a labelled polynucleotide comprising a nucleotide sequence corresponding to at least part of the gene encoding PTP-1 B and containing a mutation of at least one nucleotide, which mutation corresponds to the mutation the presence of which in the gene encoding PTP-1 B is to be detected.

In one embodiment of the present invention, the labelled polynucleotide in said test kit comprises a polynucleotide according to the present invention.

In a further embodiment of the present invention, said test kit further comprises a second labelled polynucleotide comprising a nucleotide sequence corresponding to at least part of the wild-type gene encoding PTP-1 B.

In a further embodiment of the present invention, the label substance with which the labelled polynucleotide in said kit carrying the mutation is labelled is different from the label substance with which the second labelled polynucleotide corresponding to at least part of the wild-type DNA is labelled.

In a further embodiment of the present invention, the second labelled polynucleotide in said test kit is a DNA construct.

In one embodiment, the present invention encompasses a test kit suitable for use in a method according to the present invention.

In one embodiment, the present invention encompasses a test kit according to the present invention for determining predisposition to type 2 diabetes in a subject.

In one embodiment, the present invention encompasses an isolated polypeptide obtainable by expression of a DNA construct comprising a polynucleotide according to the present invention, where said mutation gives rise to an amino acid substitution in PTP-1B. Such a DNA construct may be expressed as part of a recombinant expression vector as described above and as it is generally known in the art. In a further embodiment the amino acid substitution in said isolated polypeptide obtainable by expression of a DNA construct comprising a polynucleotide according to the present invention is a substitution of Pro to an amino acid different from Pro in the position corresponding to position 387 of the amino acid sequence shown in the Sequence Listing as SEQ ID NO: 2. In a further embodiment this amino acid substitution is a substitution of Pro to Lys in the position corresponding to position 387 of the amino acid sequence shown in the Sequence Listing as SEQ ID NO: 2. In another further embodiment said polypeptide has a degree of phosphorylation in a p34$^{cdc2}$ kinase phosphorylation assay which is significantly lower than the degree of phosphorylation of a second polypeptide encoded by a second polynucleotide which second polypeptide differs from the first polynucleotide only by not containing said mutation associated with type 2 diabetes. In a still further embodiment, said phosphorylation takes place at the serine residue in the position corresponding to corresponding to position 386 of the amino acid sequence shown in the Sequence Listing as SEQ ID NO: 2.

The present invention also encompasses an isolated polypeptide, which is a variant of PTP-1B carrying an amino acid substitution associated with type 2 diabetes and which variant is selected from the group consisting of (a) a polypeptide having an amino acid sequence which is substantially homologous to residues 1 to 435 of SEQ ID NO: 2; (b) a polypeptide which is encoded by a polynucleotide comprising a nucleic acid sequence which hybridizes under low stringency conditions with (i) nucleotides 91 to 1395 of SEQ ID NO: 1 or (ii) a subsequence of (i) of at least 100 nucleotides, (c) a variant of a polypeptide comprising an amino acid sequence of SEQ ID NO: 2 comprising a substitution, deletion, and/or insertion of one or more amino acids; (d) an allelic variant of (a) or (b); and (e) a fragment of (a), (b), (c) or (d).

The term "substantially homologous" is used herein to denote polypeptides having a sequence identity to the sequences shown in SEQ ID NO: 2 of at least about 65%, or at least about 70%, or at least about 80%, or at least about 90%, or at least about 95%, or at least about 97% while still having the function of structure of PTP-1B. Percent sequence identity is determined by conventional methods. See, for example, Altschul et al., Bull. Math. Bio. 48, 603-616 (1986) and Henikoff and Henikoff, Proc. Natl. Acad. Sci. USA 89, 10915-10919 (1992). Briefly, two amino acid sequences are aligned to optimize the alignment scores using a gap opening penalty of 10, a gap extension penalty of 1, and the "blosum 62" scoring matrix of Henikoff and Henikoff (ibid.) as shown in Table 1 (amino acids are indicated by the standard one-letter codes). The percent identity is then calculated as:

$$\frac{\text{Total number of identical matches}}{\text{[length of the longer sequence plus the number of gaps introduced into the longer sequence in order to align the two sequences]}} \times 100$$

Sequence identity of polynucleotide molecules is determined by similar methods using a ratio as disclosed above.

Substantially homologous polypeptides are characterized as having one or more amino acid substitutions, deletions or additions. These changes are preferably of a minor nature, that is conservative amino acid substitutions (see Table 2) and other substitutions that do not significantly affect the folding or activity of the protein or polypeptide; small deletions, typically of one to about 30 amino acids; and small amino- or carboxyl-terminal extensions, such as an amino-terminal methionine residue, a small linker peptide of up to about 20-25 residues, or a small extension that facilitates purification (an affinity tag), such as a poly-histidine tract, protein A (Nilsson et al., EMBO J. 4, 1075 (1985); Nilsson et al., Methods Enzymol. 198, 3, 1991), glutathione S transferase (Smith et al., Gene 67, 31 (1988), maltose binding protein (Kellerman et al., Methods Enzymol. 90, 459-463 (1982); Guan et al., Gene 67, 21-30 (1987)), thioredoxin, ubiquitin, cellulose binding protein, T7 polymerase, or other antigenic epitope or binding domain. See, in general Ford et al., Protein Expression and Purification 2, 95-107, 1991, which is incorporated herein by reference. DNAs encoding affinity tags are available from commercial suppliers (e.g., Pharmacia Biotech, Piscataway, NJ; New England Biolabs, Beverly, MA). It is readily apparent to the person skilled in the art that the present invention also encompasses polypeptides according to the present invention, which carry more than one amino acid substitution associated to type 2 diabetes or other disorders associated with type 2 diabetes, like obesity, hyperlipidemia and hypertension. Similarly, the present invention encompasses polypeptides which in addition to one or more amino acid substitutions associated with type 2 diabetes carries other amino acid substitutions of interest such as amino acid substitutions which do significantly affect the folding or activity of the polypeptide.

In addition to the 20 standard amino acids, non-standard amino acids (such as 4-hydroxyproline, 6-N-methyl lysine, 2-aminoisobutyric acid, isovaline and α-methyl serine) may be substituted for PTP-1B amino acid residues. A limited number of non-conservative amino acids, amino acids that are not encoded by the genetic code, and unnatural amino acids may be substituted for PTP-1B amino acid residues. "Unnatural amino acids" have been modified after protein synthesis, and/or have a chemical structure in their side chain(s) different from that of the standard amino acids. Unnatural amino acids can be chemically synthesized, or preferably, are commercially available, and include pipecolic acid, thiazolidine carboxylic acid, dehydroproline, 3- and 4-methylproline, and 3,3-dimethylproline.

For polynucleotides of at least 100 nucleotides in length, low, medium and high stringency conditions are defined as prehybridization and hybridization at 42°C in 5X SSPE, 0.3% SDS, 200 µg/ml sheared and denatured salmon sperm DNA, and either 25% formamide for low stringency, 35% formamide for medium stringency, or 50% formamide for high stringencies, following standard Southern blotting procedures.

A variant of a polypeptide comprising an amino acid sequence of SEQ ID NO: 2 is a polypeptide which has an amino acid sequence which is substantially similar to the amino acid sequence in SEQ ID NO: 1. Such variants may be the result of modification of a nucleic acid sequence of a polynucleotide according to the present invention which may be desirable for example for increasing the yield of the produced polypeptide or which might otherwise be desirable for handling the polypeptide. The term "substantially similar" to the amino acid sequence refers to amino acid sequences of non-naturally occurring forms of the polypeptide. These polypeptides may differ in some engineered way from PTP-1B as isolated from its native source, *e.g.*, variants that differ in specific activity, thermostability, pH optimum, or the like. The variant sequence may be constructed on the basis of the nucleic acid sequence presented as the polypeptide encoding part of SEQ ID NO: 1, *e.g.*, a subsequence thereof, and/or by introduction of nucleotide substitutions which do not give rise to another amino acid sequence of the polypeptide encoded by the nucleic acid sequence, but which corresponds to the codon usage of the host organism intended for production of the polypeptide, or by introduction of nucleotide substitutions which may give rise to a different amino acid sequence or in other ways. For a general description of nucleotide substitution, see for instance Ford et al., Protein Expression and Purification 2, 95-107 (1991).

An allelic variant denotes any of two or more alternative forms of a gene occupying the same chromosomal locus. Allelic variation arises naturally through mutation, and may result in polymorphism within populations. Gene mutations can be silent (no change in the encoded polypeptide) or may encode polypeptides having altered amino acid sequences. The allelic variant of a polypeptide is a polypeptide encoded by an allelic variant of a gene.

The polypeptides of the present invention, including full-length proteins, fragments thereof and fusion proteins, can be produced in genetically engineered host cells according to conventional techniques. Suitable host cells are those cell types that can be transformed or transfected with exogenous DNA and grown in culture, and include bacteria, fungal cells, and cultured higher eukaryotic cells. Eukaryotic cells, particularly cultured cells of multicellular organisms, are preferred. Techniques for manipulating cloned DNA molecules and introducing exogenous DNA into a variety of host cells are disclosed by Sambrook et al. ibid, and Ausubel et al. (eds.), Current Protocols in Molecular Biology, John Wiley and Sons, Inc., NY, 1987, which are incorporated herein by reference.

Polypeptides according to the present invention can be purified using fractionation and/or conventional purification methods and media. Ammonium sulfate precipitation and acid or chaotrope extraction may be used for fractionation of samples. Exemplary purification steps may include differential centrifugation, hydroxyapatite, size exclusion, such as for instance gel filtration, FPLC, ion-exchange chromatography, affinity chromatography, membrane filtration, such as for instance ultrafiltration or diafiltration, or preparative HPLC or any combinations thereof. Suitable anion exchange media include derivatized dextrans, agarose, cellulose, polyacrylamide, specialty silicas, and the like. PEI, DEAE, QAE and Q derivatives are preferred, with DEAE Fast-Flow Sepharose (Pharmacia, Piscataway, NJ) being particularly preferred. Exemplary chromatographic media include those media derivatized with phenyl, butyl, or octyl groups, such as Phenyl-Sepharose FF (Pharmacia), Toyopearl butyl 650 (Toso Haas, Montgomeryville, PA), Octyl-Sepharose (Pharmacia) and the like; or polyacrylic resins, such as Amberchrom CG 71 (Toso Haas) and the like. Suitable solid supports include glass beads, silica-based resins, cellulosic resins, agarose beads, cross-linked agarose beads, polystyrene beads, cross-linked polyacrylamide resins and the like that are insoluble under the conditions in which they are to be used. These supports may be modified with reactive groups that allow attachment of proteins by amino groups, carboxyl groups, sulfhydryl groups, hydroxyl groups and/or carbohydrate moieties. Examples of coupling chemistries include cyanogen bromide activation, N-hydroxysuccinimide activation, epoxide activation, sulfhydryl activation, hydrazide activation, and carboxyl and amino derivatives for carbodiimide coupling chemistries. These and other solid media are well known and widely used in the art, and are available from commercial suppliers. Selection of a particular method is a matter of routine design and is determined in part by the properties of the chosen support. See, for example, Affinity Chromatography: Principles & Methods, Pharmacia LKB Biotechnology, Uppsala, Sweden, 1988.

Protein refolding (and optionally reoxidation) procedures may be advantageously used. It is preferred to purify the protein to at least 80% purity, or to at least 90% purity, or to at least 95%, or to a pharmaceutically pure state, that is at least 99.9% pure with respect to contaminating macromolecules, particularly other proteins, polypeptides and nucleic acids, and free of infectious and pyrogenic agents. Preferably, a purified polypeptide is substantially free of other proteins, particularly other proteins of animal origin.

Polypeptides according to the present invention or fragments thereof may also be prepared through chemical synthesis for instance by use of solid-phase peptide synthesis.

In one embodiment the present invention encompasses an isolated polypeptide, which is a variant of PTP-1B carrying an amino acid substitution associated with type 2 diabetes and which variant is a polypeptide which is encoded by a polynucleotide comprising a nucleic acid sequence which hybridizes under medium stringency conditions with (i) nucleotides 91 to 1395 of SEQ ID NO: 1 or (ii) a subsequence of (i) of at least 100 nucleotides.

In another embodiment the present invention encompasses an isolated polypeptide, which is a variant of PTP-1 B carrying an amino acid substitution associated with type 2 diabetes and which variant is a polypeptide which is encoded by a polynucleotide comprising a nucleic acid sequence which hybridizes under high stringency conditions with (i) nucleotides 91 to 1395 of SEQ ID NO: 1 or (ii) a subsequence of (i) of at least 100 nucleotides.

In one embodiment the present invention encompasses a method for determining the ability of a composition to regulate the phosphorylation of PTP-1 B, which method comprises combining said composition with a polypeptide according to the present invention and determining the degree of phosphorylation of said polypeptide. In a further embodiment of this method the phosphorylation of said polypeptide takes place at the amino acid residue corresponding to Ser in position 386 of SEQ ID NO: 2. In another further embodiment of this method the amino acid substitution in said polypeptide according to the present invention is a substitution of Pro to an amino acid different from Pro in the position corresponding to position 387 of the amino acid sequence shown in the Sequence Listing as SEQ ID NO: 2. In a still further embodiment of this method the amino acid substitution in said polypeptide according to the present invention is a substitution of Pro to Lys in the position corresponding to position 387 of the amino acid sequence shown in the Sequence Listing as SEQ ID NO: 2. In one embodiment of the present invention said method also comprises a step, where the degree of phosphorylation of a polypeptide according to the present invention is compared to the degree of phosphorylation of a polypeptide differing from said polypeptide only in that it does not comprise said amino acid substitution associated with type 2 diabetes or other disorders associated with type 2 diabetes.

Said composition may be any type of composition, but will typically be a liquid composition such as a solution or suspension. Said composition may for instance be a composition comprising substances, which are suspected of influencing the phosphorylation of PTP-1B or may be a composition, which comprises substances, the effect of which on the phosphorylation of PTP-1 B is to be determined or other substances of interest. The compositions may also be compositions not containing such substances for use as control samples. Compositions for use in a method according to the present invention may be biological samples from subjects or cell cultures such as bodily fluids, whole cell extracts, cell culture supernatants. These samples may be treated by any means available prior to being combined with a polypeptide according to the present invention. If the composition for instance comprises a protein which is suspected of influencing the phosphorylation of PTP-1B, this composition may be purified as it is known in the art and tested by use of said method according to the invention after one, some or all of the steps of the purification for instance for identifying the specific protein in question. The compositions may also be solutions or suspensions of known substances or any other type of composition, which may be of interest for use in such a method.

The ability of the compositions to regulate the phosphorylation of PTP-1 B may arise from the ability of one or more of the substances in the composition to regulate phosphorylation of PTP-1B. Regulation of phosphorylation should be conceived as meaning any effect influencing that is resulting in a change in the phosphorylation of PTP-1B which can be measured in an assay for measuring phosphorylation, for instance in a p34$^{cdc2}$ kinase phosphorylation assay. Other substances may be involved in the regulation and it is definitely conceivable that the combination of said composition and the polypeptide according the present invention may take place in the presence of other substances, such as for instance additional polypeptides or proteins, co-activators, or substrates. The combination may also take place in the presence of solvents such as for instance buffer such as aqueous buffers as it is known in the art. Substances with the ability alone or in concert to regulate phosphorylation of PTP-1B may be proteins that are present in PTP-1 B's natural environment such as for instance kinases able to phosphorylate PTP-1 B or proteins regulating the activity of such kinases. Such proteins may for instance be identifiable by use of mutations, which suppress the effect of the amino acid substitution associated with type 2 diabetes. Such proteins may be potential drug targets for developing pharmaceuticals for treating type 2 diabetes in subjects carrying a mutation in PTP-1 B associated with type 2 diabetes. Substances with the ability alone or in concert to regulate phosphorylation of PTP-1 B may also be other types of molecules such as potential drug candidates, which act on the proteins involved in the phosphorylation of PTP-1 B or on PTP-1 B itself.

The degree of phosphorylation may be determined as described elsewhere in the present description.

In the present study we have identified a rare P387L variant of the *PTP-1B* gene that is associated with type 2 diabetes in the Danish population and impaired *in vitro* serine phosphorylation of a PTP-1 B peptide.

Using PCR-single strand conformational polymorphism (PCR-SSCP) and heteroduplex analysis cDNA of *PTP-1B* from 56 insulin resistant patients with type 2 diabetes as well as cDNA from 56 obese patients was analysed.

The analysis on cDNA revealed 5 variants: Four silent variants (NT CGA→ CGG) R199R, (NT CCC→ CCT) P303P, 3'UTR+104ins, 3'UTR+86T→ G, and one missense variant (NT CCA→ CTA) P387L. Subsequent analysis on genomic DNA revealed 4 variants: two intron variants IVS9+57C→ T, and IVS9+58G→ A and two missense variants (NT GGT→ AGT) G381S, and (NT ACG→ ATG) T420M. The prevalence of the G381S variant was 0.4% (95% Cl: -0.001 - 0.009) among 527 type 2 diabetic patients and 1.1% (95% Cl: -0.002 - 0.02) among 234 matched control subjects (p = 0.143). The prevalence of the P387L variant was 1.2% (95% Cl: 0.003 - 0.02) among 527 type 2 diabetic patients and 0% among 234 matched control subjects ($p$ = 0.013). The 3'UTR+104insG insertion variant had a prevalence of 6.9% (95% Cl: 0.05 - 0.09) among 490 type 2 diabetic patients and 8.8% (95% Cl: 0.05 - 0.13) among 221 matched control subjects ($p$ = 0.066) (Table 4). *In vitro* p34[cdc2] kinase-directed incorporation of [$Y$-$^{32}$p] ATP was reduced in a mutant peptide compared to wild type (387P 100% vs. 387L 28.4% ± 5.8, p = 0.0012).

The proline at position 387 in the N-terminal regulatory region of the protein is conserved between mouse, rat and humans and is, in the human sequence, located next to a serine residue (position 386) and is part of a consensus sequence known to be phosphorylated *in vitro* by the proline-directed kinase p34[cdc2] (Moreno S et al., Cell 61, 549-551 (1990), Kamijo Met al., Pept Res 5, 281-285 (1992)). Replacing the proline by a leucine reduced the phosphorylation of the serine by 70% in our *in vitro* studies of the relevant peptide. Testing the replacement on the NetPhos 2.0 Phosphorylation Prediction database further confirmed that the predicted likelihood of the serine 386 being a phosphorylation target is reduced from 0.884 to 0.135 (range from 0 to 1.0) when replacing proline 387 with leucine (Blom Net al., J. Mol.Biol. 294, 1351-1362 (1999)).

The search for variability in the human PTP-1 B gene also identified several additional variants of which only the silent variant P303P has previously been identified and reported to have no association with type 2 diabetes (Klupa T et al., Am. J. Human Genet 65(suppl.4), 833A (1999)). The association study of the missense variant G381S and the insertion variant 3'UTR+104insG showed no significant association with type 2 diabetes, although the 3'UTR+104insG variant shows a trend towards an association. These findings need to be tested in larger association studies.

A rare P387L variant in the PTP-1 B gene that is associated with type 2 diabetes in the examined Danish population has thus been identified. The variant peptide exhibits reduced *in vitro* serine phosphorylation. As deficiencies in the phosphorylation of PTP-1 B, especially of the serine residue in position 386, may be involved in the development of type 2 diabetes, other proteins involved in this phosphorylation may be strong candidates for acting as drug targets for drug development with the aim of treating type 2 diabetes or other disorders relating to type 2 diabetes. Such proteins could for instance be involved in the actual phosphorylation of PTP-1 B or in another modification of PTP-1 B depending on the phosphorylation status of PTP-1 B. Testing for association of the variant to diabetes in other populations as well as further studies of the effect of the variant on PTP-1 B function are needed.

**Example 1**

Research design and methods Study groups.

The primary mutation analysis was performed on cDNA derived from muscle biopsies from 56 unrelated insulin resistant type 2 diabetic patients (Hansen L et al., Hum Mol Genet 4, 1313-1320 (1995)) and on cDNA derived from subcutaneous fat biopsies from 56 unrelated obese patients (BMI range 34-56 kg/m$^2$), respectively. The association studies of the G381S, P387L and 3'UTR+104insG variants were done in 527 unrelated type 2 diabetic patients who were recruited from Steno Diabetes Centre, Denmark (58% men and 42% women), age 60 ± 11 years, and with a mean BMI of 29 ± 5 kg/m$^2$. Twenty eight percent of the patients were treated with diet, 57% with OHA, and 15% with insulin. The association studies also comprised 234 unrelated control subjects (49% men and 51% women), age 52 ± 14 years, with a mean BMI of 25 ± 4 kg/m$^2$ traced in the Central population Register, who had a normal glucose tolerance following an OGTT. All study participants were Danish by self-report. The studies were approved by the ethics committee of Copenhagen, and were carried out in accordance with the Helsinki Declaration II. Before participating in the study, informed consent was obtained from all subjects.

Mutation analyses.

A primary mutation analysis on cDNA prepared from total RNA extracted from 56 biopsies from the vastus lateralis muscle and 56 subcutaneous fat biopsies was conducted according to standard procedures (Hansen L et al., ibid.). The PTP-1 B cDNA was amplified as a primary PCR segment using primers PF and PR (Table 3) covering parts of the 5' and 3' untranslated region and the coding region (SEQ ID NO: 1, GenBank accession number M31724, published by in Chernoff J, Proc Natl Acad Sci USA 87, 2735-2739 (1990)).

Secondary PCR's were performed with incorporation of [$a$-$^{32}$p] dCTP on 2 µl of the primary PCR in a total reaction volume of 25 µl. In this way, the primary PCR was reamplified in seven overlapping secondary PCR segments ranging in size from 267 to 313 base pairs (Table 3). All PCR reactions were performed with denaturation at 95°C for 2 min followed by 95°C for 30 s, annealing for 30 s (annealing temperature for each primer set is given in Table 3), 72°C elongation for 30 s in 35 cycles, and finally an extension at 72°C for 9 min, except for the primary PCR that was elongated for 2 min. The secondary PCR samples were analysed by single-strand conformational polymorphism (PCR-SSCP) and heteroduplex analysis applying two different experimental conditions as previously described (Echwald SM, Biochem Biophys Res Commun 233, 248-252 (1997)). The variants identified were sequenced on both strands using an ABI377 automated sequencer (Perkin Elmer, Forster City, Ca, USA) or by dideoxysequencing using the Thermo Sequenase cycle sequencing kit, (USB Corporation, Cleveland, USA). All variants identified were confirmed on genomic DNA.

Genotyping

Genotyping of the P387L variant and the G381S variant was carried out by PCR amplification of a segment covering exon 9 on genomic DNA using the primers (5'-3'sense primer) TACCCATCTCTGCCCTCT and (5'-3' antisense primer) GGTAGGATTCAGTTCT-GTG ($T_{anneal}$ 56°C and $MgCl_2$ 1.5 mM) and PCR-SSCP and heteroduplex analysis. Genotyping of the 3'UTR+104insG variant was carried out by PCR amplification of a segment covering exon 10 and part of the 3'UTR region using the primers (5'-3' sense primer) GTCTGGGCTCATCTGAACTGT and (5'-3' antisense primer) GGACGGACGTTGGTTCTG ($T_{anneal}$ 58°C and $MgCl_2$ 1.5 mM) and PCR-SSCP and heteroduplex analysis.

Genotyping of the P387L variant can also be carried out by PCR amplification of a 327 base pair segment covering exon 9 on genomic DNA using the primers (5'-3'sense primer) CATCTCTGCCCTCTGATTCC and (5'-3' antisense primer) TGAGACTGGCTCAGA-TGCAC ($T_{anneal}$ 60°C and 2.0 mM $MgCl_2$). The mutation removes a *Bs*/I restriction endonuclease site at position 101 in the segment. An additional control site is present at position 290 in the segment. Thus enzyme digestion of PCR segments from wildtype carriers will produce 3 fragments: 37bp, 101bp, and 189bp whereas homozygous mutant carriers will produce only the 37bp fragment and a 290bp fragment. Heterozygous carriers will contain all four fragments. Screening is performed by adding 5 units of *Bs*/I enzyme (New England Biolabs) into 20 µl of amplified PCR product and after 4 h incubation at 55°C, products are loaded onto 3% agarose gels and visualized by staining with ethidium bromide.

Statistical analysis.

Fisher's exact test as implemented in AssoTest ver. 04a was applied to test for differences in allele frequencies between diabetic and non-diabetic subjects. $p < 0.05$ is considered significant. The Statistical Package of Social Science (SPSS) software for windows (version 10) was used to carry out descriptive analysis and analysis using a generalized linear model. The analysis included age and BMI as covariate and gender as a fixed factor. The normal distribution of the residuals was verified visually.

The Image Quant (version 5.1) was used to quantify the radioactive incorporation into wild type and mutated peptides. The wild type from each assay is set to 100 % and the mutant is counted as a percentage of the wild type. Four independent *in vitro* peptide phosphorylation assays were performed. A two-tailed distribution paired t-test was used on the log10-transformed data set from each assay. The values are expressed as mean ± SD. Standard deviation among the 4 independent *in vitro* peptide phosphorylation assays is calculated from the log10-transformed data set.

*In vitro peptide $p34^{cdc2}$ kinase phosphorylation assay.*

**Incorporation of [*y*-$^{32}$p] ATP into wild type peptide (RRRGAQAASPAKGE: 387P) and mutant peptide (RRRGAQAASLAKGE: 387L) by the $p34^{cdc2}$ kinase was performed in a final reaction mixture containing 50 µl of 1 mM of wild type (387P) or mutant peptide (387L), 100 µM ATP, at a final specific activity of 100 µCi/µmol [*y*-$^{32}$p] ATP (Amersham Pharmacia Biotech), reaction buffer containing 50 mM Tris-HCl, 10 mM $MgCl_2$, 2 mM DTT, 1 mM EGTA, 0.01% Brij 35, pH 7.5 and 0.272 unit recombinant $p34^{cdc2}$ protein kinase purchased from New England Biolabs. For negative and positive controls myelin basic protein (MBP, Sigma-Aldrich, Cat. # M2295) 0.33 µg/µl in a 50 µl reaction was used. The substrate and reaction mixtures were heated at 30° for 1 min separately before mixing and hereafter incubated at 30°C for 30 min. The reaction was stopped by the addition of 50 µl 2 x Tricine SDS sample buffer (LC1676-Novex recipe) and 2.5% 2-mercaptoethanol. The samples were subsequently heated for 2 min at 85°C and hereafter 25 µl of each sample was applied on a 16% tricine gel (Invitrogen, Novex) and electrophoresed for 2 hours at 110 volts. The tricine gels were silver stained to ensure peptide location using the protein silver stain kit, PlusOne (Amersham Pharmacia Biotech AB). After gel exposure to phosphoimager screens the radioactivity of the peptides was quantified by scanning in a Typhoon 8600, Instrument QuickStart v1.0, molecular Dynamics, Amersham Pharmacia Biotech. The peptides were synthesized utilizing Fmoc chemistry and the resin cleaved peptides were analysed by Mass Spectral Analysis (MALDI/TOF) (Research Genetics, Invitrogen). Quantification of the incorporation of [*y*-$^{32}$p] ATP into wild type and mutant peptide was examined in four independent assays ( Figure 1). The wild type from each assay is standardized to 100% and the mutant is counted as a percentage of the wild type. Standard deviation is calculated from the log10-transformed data set (** Figure 2).

## Results

Mutation analysis of the PTP-1B gene

Primary analysis of the coding region of the PTP-1 B gene including the 5'-UTR from position -42 before translation start site and the 3'UTR region to position +138 after the stop codon revealed five variants: Four silent variants (NT CGA→ CGG) R199R, (NT CCC→ CCT) P303P, one insertion variant 3'UTR+104insG, and one transversion variant 3'UTR+86T→ G, and one missense variant (NT CCA→ CTA) P387L. Furthermore, during SSCP genotyping on genomic DNA using intronic primers two missense variants (NT GGT→ AGT) G381S, and (NT ACG→ ATG) T420M, and two single intron variants IVS9+57C→ T, and IVS9+58G→ A were found. The T420M, 3'UTR+86T→ G, IVS9+57C→ T and IVS9+58G→ A variants were only found in one case each.

Association studies of the missense variants G381S, and P387L, and the insertion variant 3'UTR+104insG in type 2 diabetes.

The prevalence of the G381S, P387L and 3'UTR+104insG variants were evaluated in a case control study of type 2 diabetic patients and matched control subjects. The allelic frequency of the G381S variant was 0.4% (95% Cl: -0.001 - 0.009) in type 2 diabetic patients and in control subjects the allelic frequency was 1.1% (95% Cl: -0.002-0.02).

The allelic frequency of the P387L variant was 1.2% (95% Cl: 0.003 - 0.02) in type 2 diabetic patients. In contrast, the P387L variant was not detected in 234 glucose tolerant control subjects. To rule out the possibility of any missed homozygous P387L variants on the SSCP-gel, a heterozygous single-stranded band from a SSCP-gel was cut out and a control homozygous P387L mutation was created using PCR. The homozygous pattern on SSCP-gel was distinct from the wild type and heterozygous pattern (data not shown). Thus, the P387L variant was significantly associated with type 2 diabetes ($p$ = 0.013) (Table 4). Heterozygous carriers of the P387L variant were compared with the remaining group of type 2 diabetic wild type carriers. Phenotype and biochemical characteristics were not significantly different between the two groups (Table 5).

The allelic frequency of the 3'UTR+104insG insertion variant in type 2 diabetic patients was 6.9% (95% Cl: 0.05 - 0.09). Among control subjects the allelic frequency was 8.8% (95% Cl: 0.05 - 0.13). There was no significant association of the 3'UTR+104insG variant with type 2 diabetes ($p$ = 0.066) (Table 4). The observed genotype frequencies were in Hardy-Weinberg equilibrium. Furthermore, the 3'UTR+104insG variant was not found to be associated with BMI, fasting serum insulin or fasting plasma glucose among control subjects (data not shown).

*In vitro peptide phosphorylation assay.*

The P387L variant is placed next to a serine that is phosphorylated by the proline-directed $p34^{cdc2}$ protein kinase.

**The incorporation of [$y$-$^{32}$p] labelled radioactivity into wild type peptide (387P) versus mutant peptide (387L) was investigated in an *in vitro* kinase assay. The assay results showed significantly lower $p34^{cdc2}$ kinase proline-directed phosphorylation of the mutant compared to the wild type peptides (387P 100% vs. 387L 28.4% ± 5.8, $p$ = 0.0012) (** Figure 2).

Table 1

| | A | R | N | D | C | Q | E | G | H | I | L | K | M | F | P | S | T | W | Y | V |
|---|---|---|---|---|---|---|---|---|---|---|---|---|---|---|---|---|---|---|---|---|
| A | 4 | | | | | | | | | | | | | | | | | | | |
| R | -1 | 5 | | | | | | | | | | | | | | | | | | |
| N | -2 | 0 | 6 | | | | | | | | | | | | | | | | | |
| D | -2 | -2 | 1 | 6 | | | | | | | | | | | | | | | | |
| C | 0 | -3 | -3 | -3 | 9 | | | | | | | | | | | | | | | |
| Q | -1 | 1 | 0 | 0 | -3 | 5 | | | | | | | | | | | | | | |
| E | -1 | 0 | 0 | 2 | -4 | 2 | 5 | | | | | | | | | | | | | |

Table 1 (continued)

| | A | R | N | D | C | Q | E | G | H | I | L | K | M | F | P | S | T | W | Y | V |
|---|---|---|---|---|---|---|---|---|---|---|---|---|---|---|---|---|---|---|---|---|
| G | 0 | -2 | 0 | -1 | -3 | -2 | -2 | 6 | | | | | | | | | | | | |
| H | -2 | 0 | 1 | -1 | -3 | 0 | 0 | -2 | 8 | | | | | | | | | | | |
| I | -1 | -3 | -3 | -3 | -1 | -3 | -3 | -4 | -3 | 4 | | | | | | | | | | |
| L | -1 | -2 | -3 | -4 | -1 | -2 | -3 | -4 | -3 | 2 | 4 | | | | | | | | | |
| K | -1 | 2 | 0 | -1 | -3 | 1 | 1 | -2 | -1 | -3 | -2 | 5 | | | | | | | | |
| M | -1 | -1 | -2 | -3 | -1 | 0 | -2 | -3 | -2 | 1 | 2 | -1 | 5 | | | | | | | |
| F | -2 | -3 | -3 | -3 | -2 | -3 | -3 | -3 | -1 | 0 | 0 | -3 | 0 | 6 | | | | | | |
| P | -1 | -2 | -2 | -1 | -3 | -1 | -1 | -2 | -2 | -3 | -3 | -1 | -2 | -4 | 7 | | | | | |
| S | 1 | -1 | 1 | 0 | -1 | 0 | 0 | 0 | -1 | -2 | -2 | 0 | -1 | -2 | -1 | 4 | | | | |
| T | 0 | -1 | 0 | -1 | -1 | -1 | -1 | -2 | -2 | -1 | -1 | -1 | -1 | -2 | -1 | 1 | 5 | | | |
| W | -3 | -3 | -4 | -4 | -2 | -2 | -3 | -2 | -2 | -3 | -2 | -3 | -1 | 1 | -4 | -3 | -2 | 11 | | |
| Y | -2 | -2 | -2 | -3 | -2 | -1 | -2 | -3 | 2 | -1 | -1 | -2 | -1 | 3 | -3 | -2 | -2 | 2 | 7 | |
| V | 0 | -3 | -3 | -3 | -1 | -2 | -2 | -3 | -3 | 3 | 1 | -2 | 1 | -1 | -2 | -2 | 0 | -3 | -1 | 4 |

Table 2

| Conservative amino acid substitutions | |
|---|---|
| Basic | arginine |
| | lysine |
| | histidine |
| Acidic | glutamic acid |
| | aspartic acid |
| Polar | glutamine |
| | asparagine |
| Hydrophobic | leucine |
| | isoleucine |
| | valine |
| Aromatic | phenylalanine |
| | tryptophan |
| | tyrosine |
| Small | glycine |
| | alanine |
| | serine |
| | threonine |
| | methionine |

**Table 3**

| Segment | Primers (nt nr) 5' → 3'<br>F: Forward R: Reverse | Size (bp) | Ann.T (°C) | Conc. (mM) |
|---|---|---|---|---|
| Primary (1°) | PF: (7)-GCCTCGGGGCTAAGAGC<br>PR: (1645)-AGAGAGTACCATGCTGGCG | 1657 | 58 | 2.0 |
| 1 | 1F: (49)-CAGTGGGCCGAGAAGGA<br>1R: (292)-AACGCTAGTTTGATAAAAATGGAA | 267 | 58 | 1.5 |
| 2 | 2F: (258)-GATTAAACTACATCAAGAAGA<br>2R: (525)-CTCTGAAGATATCAAGTCATA | 288 | 54 | 1.5 |
| 3 | 3F: (484)-GAGATGATCTTTGAAGACAC<br>3R: (759)-AACCTTCTGTCTGGCTGATA | 295 | 54 | 1.5 |
| 4 | 4F: (677)-TCAAAGTCCGAGAGTCAGG<br>4R: (941)-ACTCTTCCGTGCAGGATCA | 283 | 57 | 1.5 |
| 5 | 5F: (900)-CTACCTGGCTGTGATCGAAG<br>5R: (1189)-GACACTGAAGTTAGAAGTCGGG | 311 | 59 | 1.5 |
| 6 | 6F: (1152)-AAATGCCGCACCCTACG<br>6R: (1447)-AGAGCCCACGCCCGACTA | 313 | 58 | 1.5 |
| 7 | 7F: (1368)-AAATGCCGCACCCTACG<br>7R: (1645)- AGAGAGTACCATGCTGGCG | 296 | 60 | 2.0 |

Ann.T = annealing temperature

Conc. = concentration of $MgCl_2$

Table 4

| G381S | Gly/Gly | | Gly/Ser | N | Allelic frequency (%) | 95% CI | *p*-value |
|---|---|---|---|---|---|---|---|
| Diabetic patients | 523 | | 4 | 527 | 0.4 | -0.001 - 0.009 | 0.143 |
| Control subjects | 229 | | 5 | 234 | 1.1 | -0.002 - 0.02 | |
| P387L | Pro/Pro | | Pro/Leu | N | Allelic frequency (%) | 95% CI | p-value |
| Diabetic patients | 514 | | 13 | 527 | 1.2 | 0.003 - 0.02 | 0.013 |
| Control subjects | 234 | | 0 | 234 | - | - | |
| 3'UTR+104insG | wt | he | ho | N | Allelic frequency (%) | 95% CI | p-value |
| Diabetic patients | 426 | 60 | 4 | 490 | 6.9 | 0.05 - 0.09 | 0.066 |
| Control subjects | 182 | 39 | 0 | 221 | 8.8 | 0.05 - 0.13 | |
| *p*-values are based on genotype distribution | | | | | | | |

Table 5

| | P387P | P387L | *p* |
|---|---|---|---|
| Number (men/women) | 296/218 | 11/2 | |
| Age (year) | 60 (11) | 64 (10) | 0.2 |
| Age at diagnosis (year) | 50 (11) | 60 (10) | 0.5 |
| BMI (kg/$m^2$) | 29.1 (5.2) | 27.7 (4.5) | 0.5 |
| Waist/hip ratio | 0.94 (0.009) | 0.96 (0.005) | 0.8 |
| $HbA_{1c}$(%) | 8.0 (1.6) | 8.0 (1.4) | 1.0 |
| F-p-glucose (mmol/L) | 9.7 (3.3) | 10.3 (3.5) | 0.5 |
| F-C-peptide (pmol/L) | 683 (297) | 564 (297) | 0.3 |
| Insulin (pmol/L) | 77 (57) | 57 (37) | 0.4 |
| Blood pressure | | | |
| Systolic (mm HG) | 147 (24) | 146 (23) | 0.5 |
| Diastolic (mm HG) | 84 (11) | 85 (8) | 0.6 |
| Triglycerides (mmol/L) | 2.1 (1.6) | 1.9 (0.9) | 0.7 |
| Cholesterol (mmol/L) | 5.8 (1.2) | 5.8 (0.8) | 0.7 |
| HDL (mmol/L) | 1.2 (0.3) | 1.1 (0.2) | 0.3 |
| Treatment | | | |
| Diet | 28% | 27% | |
| OHA | 57% | 64% | |
| Insulin | 15% | 9% | |

Data represent means ± (SD) or number.
*p* - values are adjusted for BMI, gender and age.
OHA; Oral hyperglycaemia agents.

## Claims

1. An isolated polynucleotide molecule comprising a nucleotide sequence encoding PTP-1B, said nucleotide sequence containing a mutation associated with type 2 diabetes of a nucleotide, or comprising a fragment of the nucleotide sequence including said mutation.

2. A polynucleotide according to claim 1 comprising a nucleotide sequence as shown in the Sequence Listing as SEQ ID NO: 1 containing a mutation associated with type 2 diabetes of at least one nucleotide or comprising a fragment of the nucleotide sequence shown in the Sequence Listing as SEQ ID NO: 1 including said mutation.

3. A polynucleotide according to claim 1 or claim 2, where said mutation gives rise to an amino acid substitution in PTP-1 B.

4. A polynucleotide according to claim 3, where a polypeptide encoded by said first polynucleotide has a degree of phosphorylation in a $p34^{cdc2}$ kinase phosphorylation assay which is significantly lower than the degree of phosphorylation of a second polypeptide encoded by a polynucleotide which differs from the first polynucleotide only by not containing said mutation associated with type 2 diabetes.

5. A polynucleotide according to claim 4, where said phosphorylation takes place at the serine residue in the position corresponding to position 386 of the amino acid sequence shown in the Sequence Listing as SEQ ID NO: 2.

**6.** A polynucleotide according to any of claims 1 to 5, where said mutation gives rise to a substitution of Pro to an amino acid different from Pro in the position corresponding to position 387 of the amino acid sequence shown in the Sequence Listing as SEQ ID NO: 2.

**7.** A polynucleotide according to any of claims 1 to 6, where said mutation corresponds to a mutation of C in position 1250 in SEQ ID NO: 1 to T.

**8.** A polynucleotide according to any of claims 1 to 7 wherein said polynucleotide is a DNA construct.

**9.** A recombinant vector, especially an expression vector, comprising a polynucleotide according to any of claims 1 to 8.

**10.** A cell line or a transgenic non-human mammal containing a polynucleotide according to any of claims 1 to 8 or a recombinant vector according to claim 9.

**11.** A cell line according to claim 10 wherein the cell line is a mammalian cell line.

**12.** A method of detecting the presence of a mutation in the gene encoding PTP-1B, the method comprising obtaining a biological sample from a subject and analysing the sample for a mutation associated with type 2 diabetes of a nucleotide in the *PTP-1B* sequence.

**13.** A method according to claim 12, wherein the sample is analysed for a mutation associated with type 2 diabetes of a nucleotide in the *PTP-1B* sequence, which mutation gives rise to an amino acid substitution in PTP-1B.

**14.** A method according to claim 12 or claim 13, wherein the sample is analysed for a mutation associated with type 2 diabetes of a nucleotide in the *PTP-1B* sequence, which mutation gives rise to a substitution of Pro to an amino acid different from Pro in the position corresponding to position 387 of the amino acid sequence shown in the Sequence Listing as SEQ ID NO: 2.

**15.** A method according to any of claims 12 to 14, wherein the sample is analysed for a mutation associated with type 2 diabetes of a nucleotide in the *PTP-1B* sequence, which mutation corresponds to a mutation of C in position 1250 in SEQ ID NO: 1 to T.

**16.** A method according to any of claims 12 to 15, wherein DNA is isolated from the sample and digested with a restriction endonuclease which cleaves DNA at the site of the mutation, and cleavage of the DNA within the gene encoding PTP-1B at this site is determined.

**17.** A method according to claim 16, wherein the restriction pattern of the DNA after digestion with the restriction endonuclease is compared to the restriction pattern obtained with a negative control comprising at least a portion of wild-type DNA encoding PTP-1B.

**18.** A method according to claim 16 or claim 17, wherein the restriction pattern of the DNA after digestion with the restriction endonuclease is compared to the restriction pattern obtained with a positive control comprising at least a portion of DNA encoding PTP-1B and containing the mutation.

**19.** A method according to claim 18, wherein the positive control comprises a polynucleotide according to claim 8.

**20.** A method according to claim any of claims 16 to 19, wherein the sample is analysed for a mutation associated with type 2 diabetes of a nucleotide in the *PTP-1B* sequence, which mutation corresponds to a mutation of C in position 1250 in SEQ ID NO: 1 to T and wherein the restriction endonuclease is one which cleaves DNA at the sequence

5' ...C C N N N N N/N N G G... 3'

3' ...G G N N/N N N N N C C... 5'

**21.** A method according to claim 20, wherein the restriction endonuclease is *Bs*/I.

**22.** A method according to any of claims 16 to 21, which further comprises amplifying the DNA isolated from the sample prior to digestion with the restriction endonuclease.

**23.** A method according to any of claims 12 to 15, wherein a biological sample is obtained from a subject, DNA is isolated from the sample, the DNA is amplified and hybridised to a labelled polynucleotide comprising a nucleotide sequence encoding PTP-1B, said nucleotide sequence containing a mutation associated with type 2 diabetes of a nucleotide, or comprising a fragment of the nucleotide sequence including said mutation, which mutation corresponds to the mutation the presence of which in the gene encoding PTP-1B is to be detected, and hybridisation of the labelled polynucleotide to the DNA is determined.

**24.** A method according to claim 23, wherein the labelled polynucleotide is a labelled polynucleotide according to any of claims 1 to 8.

**25.** A method according to claims 23 or claim 24, wherein the amplified DNA is hybridised to a second labelled polynucleotide comprising a DNA sequence corresponding to at least part of the wild-type gene encoding PTP-1B, and hybridisation of said second labelled polynucleotide to the amplified DNA is determined.

**26.** A method according to claim 25, wherein the label substance with which the labelled polynucleotide carrying the mutation is labelled is different from the label substance with which the second labelled polynucleotide corresponding to at least part of the wild-type DNA is labelled.

**27.** A method according to any of claims 12 to 26 for determining predisposition to type 2 diabetes in a subject.

**28.** A diagnostic composition for determining predisposition to type 2 diabetes in a subject, the composition comprising a polynucleotide according to any of claims 1 to 8.

**29.** A diagnostic composition for detecting the presence of a mutation in the gene encoding PTP-1B, the composition comprising a polynucleotide according to any of claims 1 to 8.

**30.** A test kit for detecting the presence of a mutation associated with type 2 diabetes in the gene encoding PTP-1B, the kit comprising

(a) a first polynucleotide comprising a nucleotide sequence corresponding to at least part of the gene encoding PTP-1B and containing a mutation of at least one nucleotide, which mutation corresponds to the mutation the presence of which in the gene encoding PTP-1B is to be detected and optionally

(b) a second polynucleotide comprising a nucleotide sequence corresponding to at least part of the wild-type gene encoding PTP-1B and/or optionally

(c) a restriction endonuclease, which cleaves DNA at the site of the mutation.

**31.** A test kit according to claim 30, wherein the first polynucleotide is a polynucleotide according to any of claims 1 to 8.

**32.** A test kit according to claim 31, wherein the first polynucleotide is a DNA construct, and where said mutation corresponds to a mutation of C in position 1250 in SEQ ID NO: 1 to T and which test kit comprises a restriction endonuclease, which cleaves DNA at the site of the mutation.

**33.** A test kit according to claim 32, wherein said restriction endonuclease cleaves DNA at the sequence

5' ...C C N N N N N/N N G G... 3'

3' ...G G N N/N N N N N C C... 5'.

**34.** A test kit according to claim 33, wherein said restriction endonuclease is *Bs*/I.

**35.** A test kit according to any of claims 30 to 34, which further comprises means for amplifying DNA.

**36.** A test kit for detecting the presence of a mutation associated with type 2 diabetes in the gene encoding PTP-1B, the kit comprising

(a) means for amplifying DNA, and

(b) a labelled polynucleotide comprising a nucleotide sequence corresponding to at least part of the gene encoding PTP-1B and containing a mutation of at least one nucleotide, which mutation corresponds to the mutation the presence of which in the gene encoding PTP-1B is to be detected.

**37.** A test kit according to claim 36, wherein the labelled polynucleotide comprises a polynucleotide according to any of claims 1 to 8.

**38.** A test kit according to claim 36 or claim 37, which comprises a second labelled polynucleotide comprising a nucleotide sequence corresponding to at least part of the wild-type gene encoding PTP-1B.

**39.** A test kit according to claim 38, wherein the label substance with which the labelled polynucleotide carrying the mutation is labelled is different from the label substance with which the second labelled polynucleotide corresponding to at least part of the wild-type DNA is labelled.

**40.** A test kit according to claim 38 or claim 39, wherein the second labelled polynucleotide is a DNA construct.

**41.** A test kit suitable for use in a method according to any of claims 12 to 26.

**42.** A test kit according to any of claims 30 to 41 for determining predisposition to type 2 diabetes in a subject.

**43.** An isolated polypeptide obtainable by expression of a DNA construct, which comprises a polynucleotide according to claim 3.

**44.** An isolated polypeptide, which is a variant of PTP-1B carrying an amino acid substitution associated with type 2 diabetes and which variant is selected from the group consisting of

(a) a polypeptide having an amino acid sequence which is substantially homologous to residues 1 to 435 of SEQ ID NO: 2;
(b) a polypeptide which is encoded by a polynucleotide comprising a nucleic acid sequence which hybridizes under low stringency conditions with (i) nucleotides 91 to 1395 of SEQ ID NO: 1 or (ii) a subsequence of (i) of at least 100 nucleotides,
(c) a variant of a polypeptide comprising an amino acid sequence of SEQ ID NO: 2 comprising a substitution, deletion, and/or insertion of one or more amino acids;
(d) an allelic variant of (a) or (b); and
(e) a fragment of (a), (b), (c) or (d).

**45.** A method for determining the ability of a composition to regulate the phosphorylation of PTP-1B, which method comprises combining said composition with a polypeptide according to claim 43 or 44 and determining the degree of phosphorylation of said polypeptide.

Figure 1

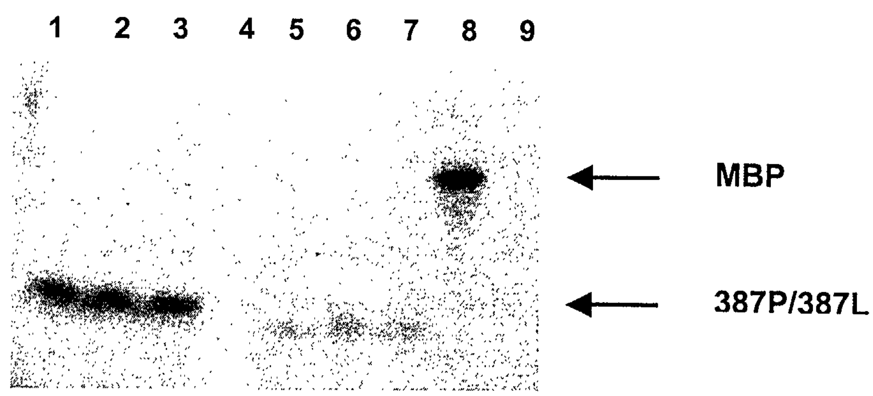

Figure 2

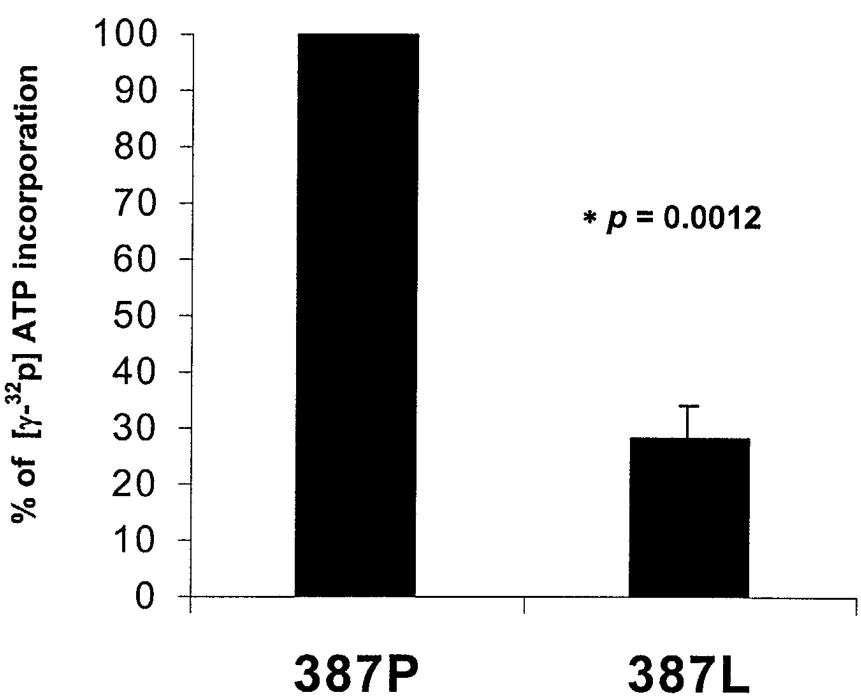
% of [γ-32p] ATP incorporation
100
90
80
70
60
50
40
30
20
10
0
* p = 0.0012
387P
387L

European Patent Office

# EUROPEAN SEARCH REPORT

Application Number
EP 01 61 0075

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int.Cl.7) |
| X | BACH H. ET AL.: "Variability in the protein tyrosine phosphatase 1b gene among Danish Type 2 diabetic and obese patients" DIABETOLOGIA, vol. 43, no. SUP1, August 2000 (2000-08), page A89 XP001021796 * abstract * | 1-45 | C12N15/55 C12N9/16 C12Q1/68 C12Q1/48 A01K67/027 |
| X | FORSELL P A L ET AL: "Genomic characterization of the human and mouse protein tyrosine phosphatase-1B genes" GENE, ELSEVIER BIOMEDICAL PRESS. AMSTERDAM, NL, vol. 260, no. 1-2, 30 December 2000 (2000-12-30), pages 145-153, XP004227533 ISSN: 0378-1119 * page 153, left-hand column, last paragraph * * page P.146, left-hand column, paragraph 2 * | 1-45 | TECHNICAL FIELDS SEARCHED (Int.Cl.7) |
| A | BYON JC ET AL: "Protein-tyrosine phosphatase-1B acts as a negative regulator of insulin signal transduction" MOLECULAR AND CELLULAR BIOCHEMISTRY, NORWELL, MA, US, vol. 182, no. 1/2, May 1998 (1998-05), pages 101-108, XP002119310 ISSN: 0300-8177 * the whole document * | | C12N C12Q |

The present search report has been drawn up for all claims

| Place of search | Date of completion of the search | Examiner |
|---|---|---|
| MUNICH | 11 October 2001 | Stolz, B |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## SEQUENCE LISTING

<110>  Novo Nordisk A/S

<120>  PTP-1b mutant

<130>  6319, KWin

<160>  2

<170>  PatentIn version 3.1

<210>  1

<211>  3246

<212>  DNA

<213>  Homo sapiens

<400>  1
gggcgggcct cggggctaag agcgcgacgc ctagagcggc agacggcgca gtgggccgag      60

aaggaggcgc agcagccgcc ctggcccgtc atggagatgg aaaaggagtt cgagcagatc     120

gacaagtccg ggagctgggc ggccatttac caggatatcc gacatgaagc cagtgacttc     180

ccatgtagag tggccaagct tcctaagaac aaaaaccgaa ataggtacag agacgtcagt     240

ccctttgacc tagtcggatt aaactacatc aagaagataa tgactatatc aacgctagtt     300

tgataaaaat ggaagaagcc caaaggagtt acattcttac ccagggccct ttgcctaaca     360

catgcggtca cttttgggag atggtgtggg agcagaaaag caggggtgtc gtcatgctca     420

acagagtgat ggagaaaggt tcgttaaaat gcgcacaata ctggccacaa aaagaagaaa     480

aagagatgat ctttgaagac acaaatttga aattaacatt gatctctgaa gatatcaagt     540

catattatac agtgcgacag ctagaattgg aaaaccttac aacccaagaa actcgagaga     600

tcttacattt ccactatacc acatggcctg actttggagt ccctgaatca ccagcctcat     660

tcttgaactt tcttttcaaa gtccgagagt cagggtcact cagcccggag cacgggcccg     720

```
ttgtggtgca ctgcagtgca ggcatcggca ggtctggaac cttctgtctg gctgatacct    780
gcctcctgct gatggacaag aggaaagacc cttcttccgt tgatatcaag aaagtgctgt    840
tagaaatgag gaagtttcgg atggggttga tccagacagc cgaccagctg cgcttctcct    900
acctggctgt gatcgaaggt gccaaattca tcatggggga ctcttccgtg caggatcagt    960
ggaaggagct ttcccacgag gacctggagc ccccacccga gcatatcccc ccacctcccc   1020
ggccacccaa acgaatcctg gagccacaca atgggaaatg cagggagttc ttcccaaatc   1080
accagtgggt gaaggaagag acccaggagg ataaagactg ccccatcaag gaagaaaaag   1140
gaagcccctt aaatgccgca ccctacggca tcgaaagcat gagtcaagac actgaagtta   1200
gaagtcgggt cgtgggggga agtcttcgag gtgcccaggc tgcctcccca gccaaagggg   1260
agccgtcact gcccgagaag gacgaggacc atgcactgag ttactggaag cccttcctgg   1320
tcaacatgtg cgtggctacg gtcctcacgg ccggcgctta cctctgctac aggttcctgt   1380
tcaacagcaa cacatagcct gaccctcctc cactccacct ccacccactg tccgcctctg   1440
cccgcagagc ccacgcccga ctagcaggca tgccgcggta ggtaagggcc gccggaccgc   1500
gtagagagcc gggccccgga cggacgttgg ttctgcacta aaacccatct tccccggatg   1560
tgtgtctcac ccctcatcct tttacttttt gccccttcca ctttgagtac caaatccaca   1620
agccattttt tgaggagagt gaaagagagt accatgctgg cggcgcagag ggaaggggcc   1680
tacacccgtc ttggggctcg ccccacccag ggctccctcc tggagcatcc caggcggcgc   1740
acgccaacag cccccccctt gaatctgcag ggagcaactc tccactccat atttatttaa   1800
acaatttttt ccccaaaggc atccatagtg cactagcatt ttcttgaacc aataatgtat   1860
taaaattttt tgatgtcagc cttgcatcaa gggctttatc aaaaagtaca ataataaatc   1920
ctcaggtagt actgggaatg gaaggctttg ccatgggcct gctgcgtcag accagtactg   1980
ggaaggagga cggttgtaag cagttgttat ttagtgatat tgtgggtaac gtgagaagat   2040
agaacaatgc tataatatat aatgaacacg tgggtattta ataagaaaca tgatgtgaga   2100
ttactttgtc ccgcttattc tcctccctgt tatctgctag atctagttct caatcactgc   2160
tcccccgtgt gtattagaat gcatgtaagg tcttcttgtg tcctgatgaa aaatatgtgc   2220
ttgaaatgag aaactttgat ctctgcttac taatgtgccc catgtccaag tccaacctgc   2280
ctgtgcatga cctgatcatt acatggctgt ggttcctaag cctgttgctg aagtcattgt   2340
cgctcagcaa tagggtgcag ttttccagga ataggcattt gctaattcct ggcatgacac   2400
tctagtgact tcctggtgag gcccagcctg tcctggtaca gcagggtctt gctgtaactc   2460
```

agacattcca agggtatggg aagccatatt cacacctcac gctctggaca tgatttaggg 2520

aagcagggac acccccccgcc ccccaccttt gggatcagcc tccgccattc caagtcaaca 2580

ctcttcttga gcagaccgtg atttggaaga gaggcacctg ctggaaacca cacttcttga 2640

aacagcctgg gtgacggtcc tttaggcagc ctgccgccgt ctctgtcccg gttcaccttg 2700

ccgagagagg cgcgtctgcc ccaccctcaa accctgtggg gcctgatggt gctcacgact 2760

cttcctgcaa agggaactga agacctccac attaagtggc tttttaacat gaaaaacacg 2820

gcagctgtag ctcccgagct actctcttgc cagcattttc acattttgcc tttctcgtgg 2880

tagaagccag tacagagaaa ttctgtggtg ggaacattcg aggtgtcacc ctgcagagct 2940

atggtgaggt gtggataagg cttaggtgcc aggctgtaag cattctgagc tggcttgttg 3000

tttttaagtc ctgtatatgt atgtagtagt ttgggtgtgt atatatagta gcatttcaaa 3060

atggacgtac tggtttaacc tcctatcctt ggagagcagc tggctctcca ccttgttaca 3120

cattatgtta gagaggtagc gagctgctct gctatatgcc ttaagccaat atttactcat 3180

caggtcatta ttttttacaa tggccatgga ataaaccatt tttacaaaaa taaaaacaaa 3240

aaaagc 3246

<210> 2

<211> 435

<212> PRT

<213> Homo sapiens

<400> 2

Met Glu Met Glu Lys Glu Phe Glu Gln Ile Asp Lys Ser Gly Ser Trp
1 5 10 15

Ala Ala Ile Tyr Gln Asp Ile Arg His Glu Ala Ser Asp Phe Pro Cys
20 25 30

Arg Val Ala Lys Leu Pro Lys Asn Lys Asn Arg Asn Arg Tyr Arg Asp
35 40 45

Val Ser Pro Phe Asp His Ser Arg Ile Lys Leu His Gln Glu Asp Asn
50 55 60

```
Asp Tyr Ile Asn Ala Ser Leu Ile Lys Met Glu Glu Ala Gln Arg Ser
65                  70                  75                  80

Tyr Ile Leu Thr Gln Gly Pro Leu Pro Asn Thr Cys Gly His Phe Trp
                85                  90                  95

Glu Met Val Trp Glu Gln Lys Ser Arg Gly Val Val Met Leu Asn Arg
            100                 105                 110

Val Met Glu Lys Gly Ser Leu Lys Cys Ala Gln Tyr Trp Pro Gln Lys
        115                 120                 125

Glu Glu Lys Glu Met Ile Phe Glu Asp Thr Asn Leu Lys Leu Thr Leu
    130                 135                 140

Ile Ser Glu Asp Ile Lys Ser Tyr Tyr Thr Val Arg Gln Leu Glu Leu
145                 150                 155                 160

Glu Asn Leu Thr Thr Gln Glu Thr Arg Glu Ile Leu His Phe His Tyr
                165                 170                 175

Thr Thr Trp Pro Asp Phe Gly Val Pro Glu Ser Pro Ala Ser Phe Leu
            180                 185                 190

Asn Phe Leu Phe Lys Val Arg Glu Ser Gly Ser Leu Ser Pro Glu His
        195                 200                 205

Gly Pro Val Val Val His Cys Ser Ala Gly Ile Gly Arg Ser Gly Thr
    210                 215                 220

Phe Cys Leu Ala Asp Thr Cys Leu Leu Leu Met Asp Lys Arg Lys Asp
225                 230                 235                 240

Pro Ser Ser Val Asp Ile Lys Lys Val Leu Leu Glu Met Arg Lys Phe
                245                 250                 255

Arg Met Gly Leu Ile Gln Thr Ala Asp Gln Leu Arg Phe Ser Tyr Leu
            260                 265                 270

Ala Val Ile Glu Gly Ala Lys Phe Ile Met Gly Asp Ser Ser Val Gln
        275                 280                 285

Asp Gln Trp Lys Glu Leu Ser His Glu Asp Leu Glu Pro Pro Pro Glu
    290                 295                 300
```

```
His Ile Pro Pro Pro Pro Arg Pro Pro Lys Arg Ile Leu Glu Pro His
305                 310                 315                 320

Asn Gly Lys Cys Arg Glu Phe Phe Pro Asn His Gln Trp Val Lys Glu
                325                 330                 335

Glu Thr Gln Glu Asp Lys Asp Cys Pro Ile Lys Glu Glu Lys Gly Ser
            340                 345                 350

Pro Leu Asn Ala Ala Pro Tyr Gly Ile Glu Ser Met Ser Gln Asp Thr
        355                 360                 365

Glu Val Arg Ser Arg Val Val Gly Gly Ser Leu Arg Gly Ala Gln Ala
    370                 375                 380

Ala Ser Pro Ala Lys Gly Glu Pro Ser Leu Pro Glu Lys Asp Glu Asp
385                 390                 395                 400

His Ala Leu Ser Tyr Trp Lys Pro Phe Leu Val Asn Met Cys Val Ala
                405                 410                 415

Thr Val Leu Thr Ala Gly Ala Tyr Leu Cys Tyr Arg Phe Leu Phe Asn
            420                 425                 430

Ser Asn Thr
        435
```